# EUROPEAN PATENT SPECIFICATION

(11) **EP 1 210 459 B1**
(45) Date of publication and mention of the grant of the patent: **11.06.2008**
(21) Application number: 00955970.9
(22) Date of filing: 01.09.2000
(51) Int. Cl.: A61B 10/00, B01L 3/00

(54) **Sample collecting device and method**
Probeentnahmevorrichtung und -verfahren
Dispositif et methode de collecte d'échantillons

(30) Priority: 03.09.1999 AU PQ265899
(43) Date of publication of application: 05.06.2002
(73) Proprietor: Genetic Solutions Pty Ltd, Indooroopilly, Queensland 4068 (AU)
(72) Inventor: ARMITAGE, Sharon, May, Forest Lake, Queensland 4078 (AU); BOWLER, Desmond, Daryl, Camp Hill, Queensland 4152 (AU); DAVIS, Gerard, Peter, Yeronga, Queensland 4103 (AU); HETZEL, David, James, Stuart, Dutton Park, Queensland 4102 (AU)
(74) Representative: Murnane, Graham John
(86) International application number: PCT/AU2000/001039
(87) International publication number: WO 2001/018239

(56) References cited:
- WO-A-00/17396
- JP-A- 10 267 761
- JP-A- 11 166 929
- US-A- 3 965 888
- US-A- 5 432 097
- US-A- 5 856 102
- US-A- 5 939 259
- US-A- 6 007 104

## Description

### TECHNICAL FIELD

The present invention is concerned with a sampling system and, more particularly, with a sampling system for the storage of biological samples for subsequent analysis.

### BACKGROUND ART

Biological samples are frequently collected in the field for later analysis for a variety of purposes. The analysis to be conducted will often be an analysis of the DNA contained in the sample in order to establish the genetic profile of the sample. Such an analysis may be conducted, for example, to verify and/or trace genetic lines in stock, to identify desirable traits in animals by identifying genetic markers for these traits or to identify the source of animal or plant material in a food product. For example, meat and meat products may be traced using DNA analysis in order to ensure that substitution of a lesser quality product has not occurred at any stage in the processing of the meat product or to identify the source of meat found to be contaminated in the marketplace.

DNA analysis for the purpose of identifying an individual organism is a well-known technique. For example, United States Patent No. 5,211,286, United States Patent No. 5,101,970 and United States Patent No. 5,856,102 describe systems for the identification of individual human beings in this way. In each case the invention is concerned with a personal identification system in which DNA-containing samples such as hair are stored in sealable plastic envelopes in a person's home to assist in their identification should the person become lost or go missing. However, each of these samples relies on the goodwill of those handling the DNA-containing materials prior to undertaking the analysis to ensure the integrity of the sample, since there is no means of avoiding tampering in the system or substitution of alternative samples. Accordingly, the only use for such systems is for an individual to store samples of their own DNA-containing material where they have control of that sample, such as in the family freezer.

Attempts have been made to ensure that the identity of meat and meat products can be traced through the production process in a variety of ways. For example, the identity of beef, pigs and poultry on a batch or consignment basis is sometimes recorded using batch/consignment numbers applied to the batch/consignment source through the slaughter process to the consumer. Indeed, in some countries e.g. the United Kingdom, Government agencies issue numeric or alpha-numeric codes to farmers who subsequently allocate such codes to each of the animals bred by them. The allocated codes are generally inscribed on ear-tags applied to the animal and recorded on a card peculiar to the animal to allow for unique identification of that animal. Various other data on an animal may also be recorded, such as the vaccination records of the animal. The information can be forwarded to a Government agency progressively or at some time just prior to slaughter of the animal. Nevertheless, this system is administratively intensive and the identification and testing records are not easily integrated.

In the slaughter process a beast is often divided at an early stage, and then sub-codes identifying each half of the beast are generated and continue to be used to identify the halves. However, further division occurs later in the butchering process, at which point it becomes impractical to continue to assign codes to each batch of meat. Accordingly, although attempts have been made to continue to identify meat using tags or labels all the way through the process, it is difficult to ensure that such tags and labels are applied accurately. Therefore, the information provided in such systems may be inaccurate and the systems are highly labour intensive and expensive. Accordingly, meat and meat products will frequently be retailed without any identification tag or label able to trace the product through the slaughter process back to the beast from which it originated.

International Application No. PCT/IE98/00021 describes a method for identifying the animal from which a meat product is derived, comprising genotyping the meat, comparing the genotype with known animal genotypes and locating any matching genotype to identify the animal from which the meat product is derived. The application of this method requires that DNA analysis be conducted of all animals and the data stored and then matched to any meat products tested. Alternatively, the samples from such beasts can be stored and then analysed later if the need arises. In either case, a library of genetic information of beasts is built up and compared to the DNA profile of meat analysed, either for routine quality assurance purposes (to trace product history to ensure, for example, that substitution of an inferior quality meat has not occurred) or, in instances where contamination of meat has been identified, so that the meat may be traced back to the trade source in an effort to identify the cause of the contamination.

The sampling system proposed in PCT/IE98/00021 is to take samples from animals in the conventional manner and then place them in an identification tube or cell which is marked with the animal tag identification code, but not secured in any way. The sample is then transferred to a laboratory for PCR analysis. The labeled tube or cell is placed in a well of a microtitre plate having a multiplicity of such wells, with each well being provided with a code matching the animal tag identification code. The analysis is conducted in the marked microtitre plate but there is no way of ensuring, aside from matching the codes manually, that the correct identification tube or cell is placed in the correct well in the microtitre plate. Thus, if only the code from the microtitre plate is used for subsequent identification, errors can occur. However, of still greater concern is the possibility that samples may be switched from one identification tube or cell to another long before such cells or tubes reach the laboratory where the analysis is conducted, since the tubes or cells are not secured. Accordingly, if a person with fraudulent intent chooses to substitute one sample for another in the samples provided for DNA analysis, this substitution will not be detectable. The present invention seeks to provide a way of ensuring that the identity of a biological sample is known with certainty when an analysis of the sample is conducted.

### DISCLOSURE OF THE INVENTION

According to a first aspect of the present invention, there is provided a sample collection device for collecting and storing a biological sample for subsequent analysis, comprising:
a base sheet arranged so that the biological sample may be positioned thereon;
a cover sheet hingedly secured to said base sheet, said cover sheet being adapted for substantially irreversible adhesive securement to said base sheet over at least a substantial portion of their facing surfaces; and
a backing sheet releasably secured to the surface of said cover sheet facing said base sheet.

According to a second aspect of the present invention, there is provided a system for the analysis of a biological sample, comprising:
a sample collection device according to the first aspect;
means for taking at least a portion of said sample for analysis together with at least the part of said storage means in which it is encased;
means for digesting said sample, or portion thereof, together with at least said part of said storage means; and
means for analysing said sample.

According to a third aspect of the present invention there is provided a method of collecting and storing a biological sample, comprising the steps of:
applying said biological sample to a base sheet having a cover sheet hingedly secured thereto, said cover sheet being adapted for substantially irreversible adhesive securement to said base sheet over at least a substantial portion of their facing surfaces and bearing a backing sheet releasably secured thereto;
removing said backing sheet; and
allowing said cover sheet to adhere substantially irreversibly to the base sheet and/or the biological sample positioned on said base sheet.

According to a fourth aspect of the invention, there is provided a method of analysing a biological sample, comprising the steps of:
collecting and storing a biological sample according to the third aspect;
taking at least a portion of said sample together with at least the part of said storage means in which it is encased;
digesting said sample, or portion thereof, together with at least said part of said storage means; and
analysing said sample.

In particular, the cover sheet may be coated with a permanent adhesive across its entire surface, and the portion of the cover sheet to which the backing sheet is not secured constitutes the hinged connection between the cover sheet and the base sheet. A backing sheet is generally releasably secured to the surface of the cover sheet in order to prevent it sticking to the base sheet before it is put to use in collecting a biological sample.

Advantageously said base sheet is printed on its reverse. A bar code may be printed on this sheet together with instructions for use of the device and/or an area to write an identification code.

Advantageously, the base sheet is a sheet of paper, typically a sheet of gloss art paper. The cover sheet is typically a clear polypropylene film and the backing sheet is a release paper.

The biological sample may be any suitable body part including animal hair, hide, buccal swabs, blood, muscle, bone, scales or the organs of an animal, or may be plant material such as leaves, stems or woody material. Body fluids including blood, saliva, semen and urine may also be sampled.

Preferably the sample is subjected to analysis to establish a DNA profile, but the analysis may be for any material contained in said sample provided that it is present in sufficient quantities for the analysis and that none of the materials in said storage means interferes with the analysis. For example, the sample may be analysed for protein or mineral content, or for the content of other materials such as carbohydrate or lipid. It may also be analysed for the presence of chemicals such as chemical contaminants e.g. pesticides in the sample. Typically, the analysis comprises amplification of the DNA contained in a sample such as animal hair using the polymerase chain reaction (PCR) followed by DNA sequencing to establish a genetic profile. The purpose of the analysis used, for example, to verify and/or trace genetic lines in stock, to identify desirable traits in animals by identifying genetic markers for these traits or to identify the source of animal or plant material in a food product. In particular, meat and meat products may be traced using DNA analysis in order to ensure that substitution of a lesser quality product has not occurred at any stage in the processing of the meat product or to identify the source of meat found to be contaminated in the marketplace.

Typically, the sample is taken for analysis by punching out at least a portion of the sample that has been collected together with that part of said storage means in which it is encased, using a conventional punching device. It will be appreciated that contamination of the sample cannot occur in this process, as may occur, for example, if a sample is transferred from one vessel to another for analysis. Moreover, the integrity of the sample is ensured since there is no possibility of accidental switching of the sample at this stage.

The sample together with the part of said storage means is digested by conventional means for analysis. In the case of DNA for PCR analysis, this may be by a conventional alkali extraction or phenol/chloroform extraction. In this step, the material making up said storage means may dissolve or partially dissolve, but at least should not interfere with development of the DNA profile.

In a particularly preferred embodiment of the invention, the device also bears a code corresponding to or linked to the animal tag identification code. This means that the sample from the animal is identified at the point of taking the sample by the same unique identifier or a different unique identifier provided the two are linked as the animal, and this unique identifier remains in physical juxtaposition with the biological sample from the time it is taken to the time the sample is analysed. Given that the storage means is tamper-evident, any tampering after collection, for example when a sample is archived, will be readily apparent to the person analysing the sample.

Typically said base sheet is adapted for a biological sample to be positioned on a first surface and has printing identifying the sample on a second surface. Typically the printing is a bar-code which encodes the animal tag identification code or the animal identification code itself. In the latter case, the code may be written into an appropriate space by the person taking the sample. Typically, the second surface also includes information as to how to use the sample collection device.

The base sheet is typically a substantially rectangular sheet of paper, hence the first surface is the obverse of said base sheet and the second surface is its reverse. Preferably, the base sheet is a gloss art paper to ensure strong adhesion, and it should not contain any chemicals which will inhibit or interfere with the analysis to be conducted. Typically it is a sheet of 150gsm A2 gloss art paper

Each substantially rectangular base sheet may be joined by a line of weakness to a substantially identical sheet in order to connect a plurality of devices in accordance with the present invention. This allows the devices to be provided to the user as a roll from which individual sample storage devices may be torn off. The cover sheet may also be joined to adjacent cover sheets by a line of weakness, in which case separation of the cover sheet from the adjacent cover sheet is also necessary in order to remove an individual sample storage device. Alternatively, although it is not preferred, only the cover sheet may be connected to adjacent cover sheet by a line of weakness.

The base sheet and the cover sheet also include an elliptical bite taken therefrom which makes it easier for the backing sheet to be removed from the cover sheet, and is also useful in lining up rolls of individual sample storage devices during printing of the roll. The cover sheet may be hingedly secured to the base sheet in any convenient manner, but is typically secured thereto through adhesive securement along a line adjacent an edge of the base sheet. The adhesive securement may be along the entire length of said first edge or along a portion of said edge.

Typically, the cover sheet is coated across its entire surface with a permanent adhesive and the backing sheet is applied to that portion of the cover sheet which is intended to encase the biological sample. The remainder of the cover sheet then adheres to the base sheet in order to hingedly secure it thereto.

The cover sheet is typically a polymeric film, preferably a clear polypropylene film.

The adhesive may be any suitable adhesive, and is typically a pressure-sensitive adhesive. It should contain no animal products so as not to introduce any foreign DNA into the analysis process.

The backing sheet is typically a release paper. In use, when the backing sheet is peeled from the cover sheet, the adhesive on the cover sheet bonds firmly and substantially irreversibly to said base sheet. Any efforts to peel the cover sheet from the base sheet would typically result in destruction of the base sheet and/or the cover sheet, or at least in sufficient mutilation of the two for the attempt to tamper with a sample to be apparent.

An absorbent material may be secured on the front surface of said base sheet. This makes collection of body fluids easier as a quantity of these may be absorbed by the absorbent layer. Typically the absorbent layer is blotting paper.

Devices in accordance with the present invention may also be supplied together with a sampling device for sampling animal tissue.

The sample collection device may be supplied as a part of a kit which further comprises a sampling device. The sampling device preferably takes a consistent and reproducible sample from animals whilst simultaneously avoiding any cross-contamination of tissue. The nature of the sampling device will be well understood by the person skilled in the art, but is typically forceps or pliers. The kit may also include instructions for use of the sample collection device.

### BRIEF DESCRIPTION OF THE DRAWINGS

Preferred embodiments of the present invention will now be described, by way of example only, with reference to the accompanying drawings, in which:
FIG. 1 is a bottom plan view of a device for storing a biological sample in accordance with the present invention;
FIG. 2 is a cross-section through a device for storing a biological sample in accordance with the present invention;
FIG. 3a is a flowchart illustrating the manner in which a device for storage of a biological sample in accordance with the present invention is prepared for use;
FIG. 3b is a flowchart illustrating the subsequent application of a biological sample to said device;
FIG. 3c is a flowchart illustrating the manner in which a portion of said sample is taken for analysis; and
FIG. 4 shows a device for storing a sample of a body fluid in accordance with the present invention.

### BEST MODE FOR CARRYING OUT THE INVENTION

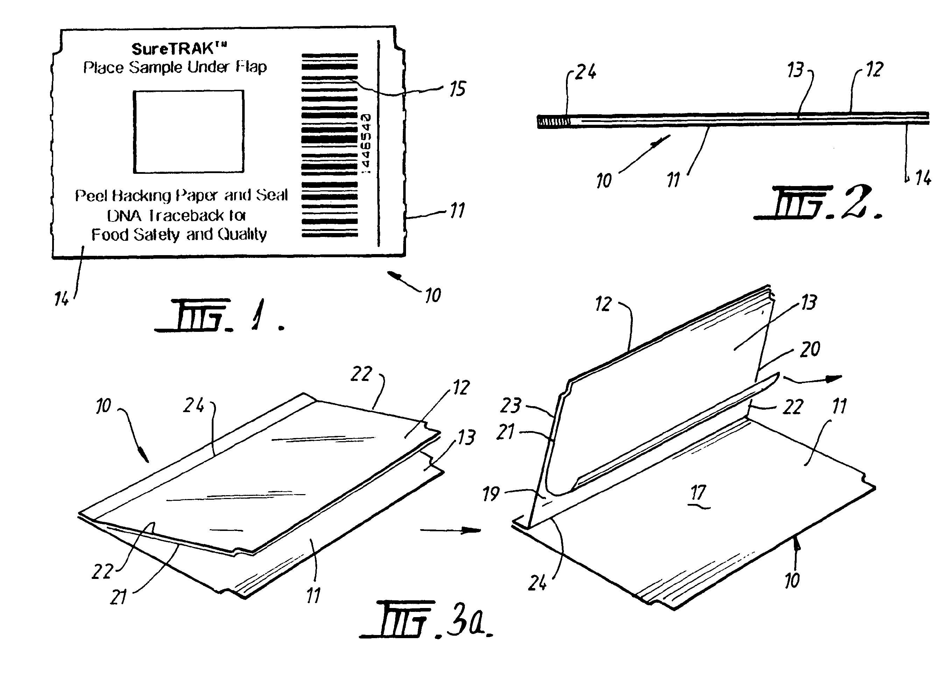

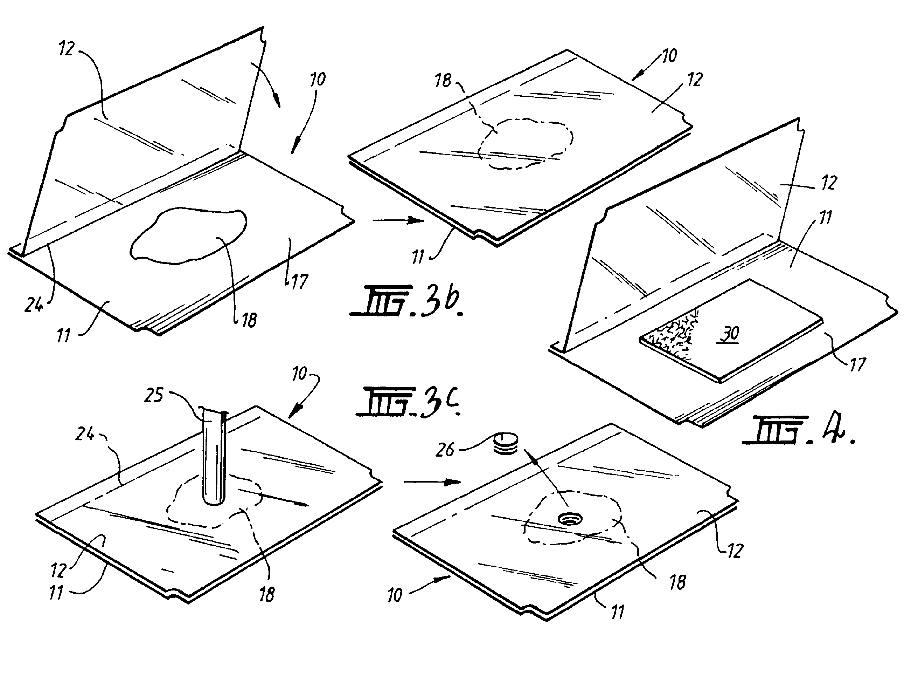

A sample storage device 10 in accordance with the present invention, as best seen in FIG. 2 and the first frame of FIG. 3a, comprises a base sheet 11 arranged so that the biological sample may be positioned thereon, a cover sheet 12 hingedly secured to the base sheet 11 and having a backing sheet 13 releasably secured thereto. The base sheet 11 is printed on its reverse 14, which contains a bar-code 15 and also a space for writing an animal tag identification code where the sampler does not have facilities for reading a bar-code. In addition, the reverse 14 of the base sheet 11 contains instructions for use of the device, as will be discussed below in relation to FIG.s 3a-3c. The base sheet 11 is a sheet of 150 gsm A2 gloss art paper adapted to receive a biological sample on its obverse surface 17, as best seen in the first frame of FIG. 3b, where a biological sample 18 has been deposited thereon. It also adheres substantially irreversibly to the cover sheet 12 when the backing sheet 13 is removed therefrom and the two are brought together.

As best seen in FIG. 3a, the cover sheet 12 is hingedly secured to the base sheet 11. In fact, the surface 19 of the cover sheet 12 facing base sheet 11 is completely covered with adhesive and backing sheet 13 is releasably secured over a portion only of the cover sheet 12. It will be appreciated that backing sheet 13 is made of a release paper and so can be easily peeled off cover sheet 12, but the adhesive on cover sheet 12 bonds substantially irreversibly to base sheet 11. This means that the portion of the cover sheet 12 which is not covered by backing sheet 13 bonds strongly to base sheet 11. Accordingly, by leaving a region of cover sheet 12 uncovered by backing sheet 13, the cover sheet 12 can be hingedly secured to base sheet 11. In this case, the backing sheet 13 is substantially rectangular in shape and corresponds in size to the size of the cover sheet 12, which is also substantially rectangular in shape, except that the length of sides 20, 21 is slightly lesser than the sides 22, 23 of cover sheet 12. Hence a small portion of cover sheet 12 adjacent an edge is left exposed, and so adheres to base sheet 11 to form a hinged connection along line 24.

As best seen in FIG. 4, a square of blotting paper 30 may be bonded to the obverse surface 17 of the base sheet 11. Samples of body fluids such as blood, saliva, semen and urine may be deposited on the blotting paper and will be absorbed.

In use, a person taking a biological sample would read the instructions on the reverse 14 of the base sheet 11 and follow these. Accordingly, that person would be directed to peel back the backing sheet 13 in the manner shown in FIG. 3a and so expose the adhesive on cover sheet 12. This person would then place biological sample 18 centrally on the obverse surface 17 of base sheet 11 and allow the cover sheet 13 to collapse onto the biological sample 18 and base sheet 11 so as to adhere to them. This is best seen in FIG. 3b. Having done this, the biological sample can be archived or sent immediately for analysis. At all times, the bar-code or animal tag identification code written on the back is in physical juxtaposition with the sample, which is encased in the sample storage device 10. If one were to attempt to remove the sample by peeling back cover sheet 13 from base sheet 11 damage to one or both sheets would occur, and the attempt to tamper with the integrity of the sample would be noted by a person subsequently conducting an analysis of the sample.

When analysis of the sample is to be conducted, a punch 25 is employed to punch a hole through the centre of biological sample 18 to create sub-sample 26. It will be appreciated that the punch removes the biological sample together with those portions of both the base sheet 11 and cover sheet 13 which encase it. The biological sample 18 does not need to be removed from the sample storage device 10 prior to analysis, hence the possibility of cross-contamination is minimised and the opportunity for tampering with the sample or substitution with another sample is limited even at the analysis stage. The sub-sample 26 that is punched out will immediately be placed in an appropriate vessel for digestion and subsequent analysis in the conventional manner.

The results of the analysis can then be matched to the animal tag identification code and/or bar-code to add to the information compiled on the beast from which the sample came. This allows for unequivocal identification of the genetic identity of the beast and so allows for comparison of a subsequent DNA analysis of a meat sample with these records to identify the source of any single piece of meat. In turn, this allows an audit line to be established to ensure that substitution of meat or meat products has not occurred and allows a source of contamination to be identified through tracing the contaminated meat back through the slaughter process to a particular beast.

The results of the analysis may also be used to verify and/or trace genetic lines in stock. Thus, the purported blood line of an animal may be checked by comparing the DNA profile compiled for the animal to the records established for other animals. Where the particular animal tested has a desirable trait, the results of the test may be used to identify genetic markers for this trait. Thereafter, animals bearing this genetic marker may be selected for when breeding in an endeavour to establish the desirable trait widely within a breed.

Alternatively, the analysis may be a chemical analysis to establish the composition of the biological sample. For example, the sample may be analysed for protein or mineral content, or for the content of other materials such as carbohydrate or lipid. The analysis may also be for the presence of chemicals such as chemical contaminants. For example, the presence of trace levels of pesticides in a sample can be detected and then the contamination traced back to its source.

### EXAMPLE 1

A biological sample collected and stored in accordance with the present invention may then be subjected to PCR analysis to obtain a DNA profile using the following method:

### Alkali Extraction Method

A hole is punched through the centre of a sample storage device in accordance with the present invention in the region where the biological sample is located. Thus, a sub-sample is created which contains a portion of the biological sample together with that part of the sample storage device in which it is encased. This material is then placed in a 0.2µl tube or well of a 96 well microtitre plate. The tube or plate is centrifuged briefly so that the sub-sample collects into the bottom of the tube or a well of the plate. 50µl of a 200mM sodium hydroxide solution is added and the mixture is incubated at 95°C for minutes. The contents of the tube or well are mixed two to three times during the incubation by quickly removing the tube or plate from the heating block and tapping several times. The mixture is then briefly centrifuged to bring down any condensation on the lids of the tube or plate. Thereafter, 50µl of a solution containing 200mM HCl, 100mM Tris.HCl, pH 8.5 is added and the mixture mixed briefly prior to centrifuging for two minutes at 13000rpm. In the next step, 80µl of the supernatant is transferred to a fresh tube/plate and diluted with 100µl sterile MilliQ H₂O. The solution is stored at -20°C for subsequent use of 1-2µl in PCR.

### Amplification and Analysis

The PCR techniques employed are conventional, and well understood by the person skilled in the art. Generally, the process involves a repetitive series of thermal cycles involving template denaturation, primer annealing and extension of the annealed primers by Taq DNA polymerase, with the result that there is an exponential accumulation of specific short DNA sequences. These DNA sequences are characteristic of the beast from which the sample was taken, and typically contain length variation at DNA sequence repeats or microsatellites which allow identification of the beast. In particular, microsatellite loci peculiar to the species of animal being tested can be amplified and analysed using the PCR process. Suitable primers are well known and, for example, are contained in the cattle paternity bovine PCR typing kit sold by Perkin Elmer under the name STOCKMARKS. This kit incorporates fluorescent tagged primers specific to eleven microsatellite loci useful in identifying cattle as well as unlabeled primers, polymerase, reference bovine DNA, dNTPs and buffers necessary to test the animals at these loci. The kit describes the procedures for conducting the analysis which are, in any event, well understood by the person skilled in the art.

The amplified product may then be subjected to a DNA fragment analysis on a suitable DNA analysis system, the likes of which are commercially available. The DNA profiles thus obtained are unique and unequivocally linked to the beast from which the sample is obtained through the audit trail described above. The genetic profile of a tissue sample subsequently obtained can be searched on a database of these genetic profiles to locate a match. Therefore, the original animal from which a tissue sample derived can be identified.

Throughout this specification and the claims, the words "comprise", "comprises" and "comprising" are used in a non-exclusive sense, except where the context requires otherwise.

Variations and modifications of this device will be apparent to the person skilled in the art, and those variations and modifications are within the scope of the present invention.

### INDUSTRIAL APPLICABILITY

The present invention ensures the integrity of biological samples taken in the field and analysed subsequently in a laboratory. Thus analysis of the samples may be conducted in order to establish a genetic profile of the sample or to ascertain its composition with the assurance that the sample has not been tampered with or modified in transit. This means that an analysis to verify and/or trace genetic lines in stock, to identify desirable traits in animals by identifying genetic markers for those traits or to identify the source of animal or plant material in a food product can be done with assurance that the results are accurate. Likewise, the samples may be analysed for a protein or mineral content, or for the content of other materials such as carbohydrate or lipid and the results may be assured. In particular, it may be analysed for the presence of chemical contaminants with the assurance that the sample has not been modified in any way in transit.

## Claims

1. A sample collection device (10) for collecting and storing a biological sample (18) for subsequent analysis, comprising:
a base sheet (11) arranged so that the biological sample (18) may be positioned thereon;
a cover sheet (12) hingedly secured to said base sheet (11), said cover sheet (12) being adapted for substantially irreversible adhesive securement to said base sheet (11) over at least a substantial portion of their facing surfaces; and
a backing sheet. (13) releasably secured to the surface of said cover sheet (12) facing said base sheet (11).

2. A device as claimed in claim 1 wherein the cover sheet (12) is coated with a permanent adhesive across its entire surface (14), and the portion of the cover sheet (12) to which the backing sheet (13) is not secured constitutes the hinged connection (24) between the cover sheet (12) and the base sheet (11).

3. A device as claimed in claim 2 wherein the adhesive is a pressure-sensitive adhesive.

4. A device as claimed in any one of claims 1, 2 or 3 wherein print is on the reverse (14) of said base sheet (11).

5. A device as claimed in claim 4 wherein a bar code (15) is printed on the reverse (14) of said base sheet (11).

6. A device as claimed in any one of claims 1 or 2 to 5 wherein the base sheet (11) is paper.

7. A device as claimed in claim 6 wherein the base sheet (11) is gloss art paper.

8. A device as claimed in any one of claims 1 or 2 to 7 wherein the cover sheet (12) is a clear polypropylene film.

9. A device as claimed in any one of claims of 1 or 2 to 8 wherein the backing sheet (13) is a release paper.

10. A system for the analysis of a biological sample, comprising:
a sample collection device as claimed in any one of claims 1 to 9;
means for taking at least a portion of said sample for analysis together with at least the part of said storage means in which it is encased;
means for digesting said sample, or portion thereof, together with at least said part of said storage means; and
means for analysing said sample.

11. A system as claimed in claim 10 wherein a hole punch (25) takes a portion of said sample for analysis together with that part of the storage means in which it is encased.

12. A system as claimed in claim 10 or claim 11 wherein said sample is digested in an alkali extraction.

13. A system as claimed in any one of claims 10 to 12 wherein said sample is subjected to amplification by PCR and then DNA sequencing.

14. A method of collecting and storing a biological sample, comprising the steps of:
applying said biological sample (18) to a base sheet (11) having a cover sheet (12) hingedly secured thereto, said cover sheet (12) being adapted for substantially irreversible adhesive securement to said base sheet (11) over at least a substantial portion of their facing surfaces and bearing a backing sheet (13) releasably secured thereto;
removing said backing sheet (13); and
allowing said cover sheet (12) to adhere substantially irreversibly to the base sheet (11) and/or the biological sample (18) positioned on said base sheet (11).

15. A method as claimed in claim 14 wherein the cover sheet (12) is coated with a permanent adhesive across its entire surface (14), and the portion of the cover sheet (12) to which the backing sheet (13) is not secured constitutes the hinged connection (24) between the cover sheet (12) and the base sheet (11).

16. A method as claimed in claim 15 wherein the adhesive is a pressure-sensitive adhesive.

17. A method as claimed in any one of claims 14, 15 or 16 wherein print is on the reverse (14) of said base sheet (11).

18. A method as claimed in claim 17 wherein a bar code (15) is printed on the reverse (14) of said base sheet (11).

19. A method as claimed in any one of claims 14 or 15 to 18 wherein the base sheet (11) is paper.

20. A method as claimed in claim 19 wherein the base sheet (11) is gloss art paper.

21. A method as claimed in any one of claims 14 or 15 to 20 wherein the cover sheet (12) is a clear polypropylene film.

22. A method as claimed in any one of claims of 14 or 15 to 21 wherein the backing sheet (13) is a release paper.

23. A method as claimed in any one of claims 14 to 22 wherein said biological sample is stored for an extended period of time.

24. A method of analysing a biological sample, comprising the steps of:
collecting and storing a biological sample (18) according to any one of claims 14 to 22;
taking at least a portion of said sample (18) together with at least the part of said storage means in which it is encased;
digesting said sample, or portion thereof, together with at least said part of said storage means; and
analysing said sample.

25. A method as claimed in claim 24 wherein a portion (26) of said sample (18) is punched out of the sample collection device with a hole punch (25).

26. A method as claimed in of claim 24 or claim 25 wherein said sample is digested in an alkali extraction.

27. A method according to any one of claims 24 to 26 wherein said sample is subjected to amplification by PCR and then DNA sequencing.

## Patentansprüche

1. Eine Probensammeivorrichtung (10) zum Sammeln und Aufbewahren einer biologischen Probe (18) zur nachfolgenden Analyse, die Folgendes beinhaltet:
ein Bodenblatt (11), das so angeordnet ist, dass die biologische Probe (18) darauf positioniert werden kann;
ein Deckblatt (12), das klappbar an dem Bodenblatt (11) gesichert ist, wobei das Deckblatt (12) an eine im Wesentlichen irreversible Klebesicherung an dem Bodenblatt (11) über mindestens einen wesentlichen Abschnitt ihrer einander zugewendeten Oberflächen angepasst ist; und
ein Rückblatt (13), das abnehmbar an der dem Bodenblatt (11) zugewendeten Oberfläche des Deckblatts (12) gesichert ist.

2. Vorrichtung gemäß Anspruch 1, wobei das Deckblatt (12) über seine gesamte Oberfläche (14) mit einem permanenten Klebemittel beschichtet ist und der Abschnitt des Deckblatts (12), an dem das Rückblatt (13) nicht gesichert ist, die klappbare Verbindung (24) zwischen dem Deckblatt (12) und dem Bodenblatt (11) ausmacht.

3. Vorrichtung gemäß Anspruch 2, wobei das Klebemittel ein Haftklebemittel ist.

4. Vorrichtung gemäß einem der Ansprüche 1, 2 oder 3, wobei sich auf der Rückseite (14) des Bodenblatts (11) ein Druckmuster befindet.

5. Vorrichtung gemäß Anspruch 4, wobei auf die Rückseite (14) des Bodenblatts (11) ein Strichcode (15) gedruckt ist.

6. Vorrichtung gemäß einem der Ansprüche 1 oder 2 bis 5, wobei das Bodenblatt (11) aus Papier ist.

7. Vorrichtung gemäß Anspruch 6, wobei das Bodenblatt (11) aus Glanzkunstdruckpapier Ist.

8. Vorrichtung gemäß einem der Ansprüche 1 oder 2 bis 7, wobei das Deckblatt (12) ein durchsichtiger Polypropylenfilm ist.

9. Vorrichtung gemäß einem der Ansprüche 1 oder 2 bis 8, wobei das Rückblatt (13) ein Trennpapier ist.

10. Ein System zur Analyse einer biologischen Probe, das Folgendes beinhaltet:
eine Probensammelvorrichtung gemäß einem der Ansprüche 1 bis 9;
ein Mittel zum Entnehmen von mindestens einem Abschnitt der Probe zur Analyse zusammen mit mindestens dem Teil des Aufbewahrungsmittels, in dem er eingeschlossen ist;
ein Mittel zum Verdauen der Probe oder eines Abschnitts davon zusammen mit mindestens dem Teil des Aufbewahrungsmittels; und
ein Mittel zum Analysieren der Probe.

11. System gemäß Anspruch 10, wobei eine Lochstanze (25) einen Abschnitt der Probe zusammen mit dem Teil des Aufbewahrungsmittels, in dem er eingeschlossen ist, zur Analyse entnimmt.

12. System gemäß Anspruch 10 oder Anspruch 11, wobei die Probe in einer Alkaliextraktion verdaut wird.

13. System gemäß einem der Ansprüche 10 bis 12, wobei die Probe einer Vervielfältigung mittels PCR und dann einer DNA-Sequenzierung unterzogen wird.

14. Ein Verfahren zum Sammeln und Aufbewahren einer biologischen Probe, das die folgenden Schritte beinhaltet:
Auflegen der biologischen Probe (18) auf ein Bodenblatt (11), das ein Deckblatt (12) aufweist, welches daran klappbar gesichert ist, wobei das Deckblatt (12) an eine im Wesentlichen irreversible Klebesicherung an dem Bodenblatt (11) über mindestens einen wesentlichen Abschnitt ihrer einander zugewendeten Oberflächen angepasst ist und ein Rückblatt (13) trägt, das daran abnehmbar gesichert ist;
Entfernen des Rückblatts (13); und
Ermöglichen, dass das Deckblatt (12) im Wesentlichen irreversibel an dem Bodenblatt (11) und/oder der biologischen Probe (18), die auf dem Bodenblatt (11) positioniert ist, klebt.

15. Verfahren gemäß Anspruch 14, wobei das Deckblatt (12) über seine gesamte Oberfläche (14) mit einem permanenten Klebemittel beschichtet ist und der Abschnitt des Deckblatts (12), an dem das Rückblatt (13) nicht gesichert ist, die klappbare Verbindung (24) zwischen dem Deckblatt (12) und dem Bodenblatt (11) ausmacht.

16. Verfahren gemäß Anspruch 15, wobei das Klebemittel ein Haftklebemittel ist.

17. Verfahren gemäß einem der Ansprüche 14, 15 oder 16, wobei sich auf der Rückseite (14) des Bodenblatts (11) ein Druckmuster befindet.

18. Verfahren gemäß Anspruch 17, wobei auf die Rückseite (14) des Bodenblatts (11) ein Strichcode (15) gedruckt ist.

19. Verfahren gemäß einem der Ansprüche 14 oder 15 bis 18, wobei das Bodenblatt (11) aus Papier ist.

20. Verfahren gemäß Anspruch 19, wobei das Bodenblatt (11) aus Glanzkunstdruckpapier ist.

21. Verfahren gemäß einem der Ansprüche 14 oder 15 bis 20, wobei das Deckblatt (12) ein durchsichtiger Polypropylenfilm ist.

22. Verfahren gemäß einem der Ansprüche 14 oder 15 bis 21, wobei das Rückblatt (13) ein Trennpapier ist.

23. Verfahren gemäß einem der Ansprüche 14 bis 22, wobei die biologische Probe über einen längeren Zeitraum aufbewahrt wird.

24. Ein Verfahren zum Analysieren einer biologischen Probe, das die folgenden Schritte beinhaltet:
Sammeln und Aufbewahren einer biologischen Probe (18) gemäß einem der Ansprüche 14 bis 22;
Entnehmen von mindestens einem Abschnitt der Probe (18) zusammen mit mindestens dem Teil des Aufbewahrungsmittels, in dem er eingeschlossen ist;
Verdauen der Probe oder eines Abschnitts davon zusammen mit mindestens dem Teil des Aufbewahrungsmittels; und
Analysieren der Probe.

25. Verfahren gemäß Anspruch 24, wobei ein Abschnitt (26) der Probe (18) mit einer Lochstanze (25) aus der Probensammelvorrichtung ausgestanzt wird.

26. Verfahren gemäß Anspruch 24 oder Anspruch 25, wobei die Probe in einer Alkaliextraktion verdaut wird.

27. Verfahren gemäß einem der Ansprüche 24 bis 26, wobei die Probe einer Vervielfältigung mittels PCR und dann einer DNA-Sequenzierung unterzogen wird.

## Revendications

1. Un dispositif de recueil d'échantillon (10) pour recueillir et stocker un échantillon biologique (18) destiné à être analysé ultérieurement, comprenant :
une feuille de base (11) disposée de façon à ce que l'échantillon biologique (18) puisse être positionné sur celle-ci
une feuille de couverture (12) fixée par charnière sur ladite feuille de base (11), ladite feuille de couverture (12) étant adaptée pour être fixée par adhésif de façon substantiellement irréversible sur ladite feuille de base (11) par-dessus au moins une portion substantielle de leurs surfaces se faisant face; et
une feuille de doublage (13) fixée de façon libérable sur la surface de ladite feuille de couverture (12) faisant face à ladite feuille de base (11).

2. Un dispositif tel que revendiqué dans la revendication 1 dans lequel la feuille de couverture (12) est enduite d'un adhésif permanent sur sa surface tout entière (14), et la portion de la feuille de couverture (12) sur laquelle la feuille de doublage (13) n'est pas fixée constitue le raccord à charnière (24) entre la feuille de couverture (12) et la feuille de base (11).

3. Un dispositif tel que revendiqué dans la revendication 2 dans lequel l'adhésif est un adhésif sensible à la pression.

4. Un dispositif tel que revendiqué dans n'importe laquelle des revendications 1, 2 ou 3 dans lequel une impression se trouve sur le verso (14) de ladite feuille de base (11).

5. Un dispositif tel que revendiqué dans la revendication 4 dans lequel un code barres (15) est imprimé sur le verso (14) de ladite feuille de base (11).

6. Un dispositif tel que revendiqué dans n'importe laquelle des revendications 1 ou 2 à 5 dans lequel la feuille de base (11) est du papier.

7. Un dispositif tel que revendiqué dans la revendication 6 dans lequel la feuille de base (11) est du papier couché lustré.

8. Un dispositif tel que revendiqué dans n'importe laquelle des revendications 1 ou 2 à 7 dans lequel la feuille de couverture (12) est un film en polypropylène transparent.

9. Un dispositif tel que revendiqué dans n'importe laquelle des revendications 1 ou 2 à 8 dans lequel la feuille de doublage (13) est un papier libérable.

10. Un système destiné à l'analyse d'un échantillon biologique, comprenant :
un dispositif de recueil d'échantillon tel que revendiqué dans n'importe laquelle des revendications 1 à 9 ;
un moyen pour prélever au moins une portion dudit échantillon destiné à être analysé de pair avec au moins la partie dudit moyen de stockage dans lequel il est enfermé ;
un moyen pour digérer ledit échantillon, ou ladite portion de celui-ci, de pair avec au moins ladite partie dudit moyen de stockage ; et
un moyen pour analyser ledit échantillon.

11. Un système tel que revendiqué dans la revendication 10 dans lequel un emporte-pièce (25) prélève une portion dudit échantillon destiné à être analysé de pair avec la partie du moyen de stockage dans lequel il est enfermé.

12. Un système tel que revendiqué dans la revendication 10 ou la revendication 11 dans lequel ledit échantillon est digéré dans une extraction alcaline,

13. Un système tel que revendiqué dans n'importe laquelle des revendications 10 à 12 dans lequel ledit échantillon est soumis à l'amplification par la PCR et ensuite au séquençage de l'ADN.

14. Une méthode de recueil et de stockage d'un échantillon biologique, comprenant les étapes de :
appliquer ledit échantillon biologique (18) sur une feuille de base (11) ayant une feuille de couverture (12) fixée par charnière sur celle-ci, ladite feuille de couverture (12) étant adaptée pour être fixée par adhésif de façon substantiellement irréversible sur ladite feuille de base (11) par-dessus au moins une portion substantielle de leurs surfaces se faisant face et portant une feuille de doublage (13) fixée de façon libérable sur celle-ci ;
retirer ladite feuille de doublage (13) ; et
permettre à ladite feuille de couverture (12) d'adhérer de façon substantiellement irréversible à la feuille de base (11) et / ou à l'échantillon biologique (18) positionné sur ladite feuille de base (11).

15. Une méthode telle que revendiquée dans la revendication 14 dans laquelle la feuille de couverture (12) est enduite d'un adhésif permanent sur sa surface tout entière (14), et la portion de la feuille de couverture (12) sur laquelle la feuille de doublage (13) n'est pas fixée constitue le raccord à charnière (24) entre la feuille de couverture (12) et la feuille de base (11).

16. Une méthode telle que revendiquée dans la revendication 15 dans laquelle l'adhésif est un adhésif sensible à la pression.

17. Une méthode telle que revendiquée dans n'importe laquelle des revendications 14, 15 ou 16 dans laquelle une impression se trouve sur le verso (14) de ladite feuille de base (11).

18. Une méthode telle que revendiquée dans la revendication 17 dans laquelle un code barres (15) est imprimé sur le verso (14) de ladite feuille de base (11).

19. Une méthode telle que revendiquée dans n'importe laquelle des revendications 14 ou 15 à 18 dans laquelle la feuille de base (11) est du papier.

20. Une méthode telle que revendiquée dans la revendication 19 dans laquelle la feuille de base (11) est du papier couché lustré.

21. Une méthode telle que revendiquée dans n'importe laquelle des revendications 14 ou 15 à 20 dans laquelle la feuille de couverture (12) est un film en polypropylène transparent.

22. Une méthode telle que revendiquée dans n'importe laquelle des revendications 14 ou 15 à 21 dans laquelle la feuille de doublage (13) est un papier de libération.

23. Une méthode telle que revendiquée dans n'importe laquelle des revendications 14 à 22 dans laquelle ledit échantillon biologique est stocké pendant une période de temps étendue.

24. Une méthode d'analyse d'un échantillon biologique, comprenant les étapes de :
recueillir et stocker un échantillon biologique (18) selon n'importe laquelle des revendications 14 à 22 ;
prélever au moins une portion dudit échantillon (18) de pair avec au moins la partie dudit moyen de stockage dans lequel il est enfermé ;
digérer ledit échantillon, ou ladite portion de celui-ci, de pair avec au moins ladite partie dudit moyen de stockage ; et
analyser ledit échantillon.

25. Une méthode telle que revendiquée dans la revendication 24 dans laquelle une portion (26) dudit échantillon (18) est découpée dans le dispositif de recueil d'échantillon avec un emporte-pièce (25).

26. Une méthode telle que revendiquée dans la revendication 24 ou la revendication 25 dans laquelle ledit échantillon est digéré dans une extraction alcaline.

27. Une méthode selon n'importe laquelle des revendications 24 à 26 dans laquelle ledit échantillon est soumis à l'amplification par la PCR et ensuite au séquençage de l'ADN.
